# EUROPEAN PATENT APPLICATION

(11) **EP 3 940 074 A1**
(43) Date of publication of application: **19.01.2022**
(21) Application number: 21189592.5
(22) Date of filing: 28.07.2017
(51) Int. Cl.: C12N 15/09, C12N 15/10, C12N 15/11, C12N 15/113, C12P 19/34, C12Q 1/68, C12N 9/12, C12Q 1/6853

(54) **METHODS AND COMPOSITIONS FOR PREVENTING CONCATEMERIZATION DURING TEMPLATE- SWITCHING**

(30) Priority: 29.07.2016 US 201662368656 P; 03.08.2016 US 201662370469 P; 03.08.2016 US 201662370501 P; 18.08.2016 US 201615240166; 18.08.2016 US 201662376493 P
(62) Divisional of application: 17835382.7
(71) Applicant: NEW ENGLAND BIOLABS, INC., Ipswich, MA 01938-2723 (US)
(72) Inventor: GUAN, Shengxi, Ipswich, MA 01938-2723 (US); EVANS, Thomas, C., Jr., Ipswich, MA 01938-2723 (US); NICHOLS, Nicole, Ipswich, MA 01938-2723 (US); BEI, Yanxia, Ipswich, MA 01938-2723 (US)
(74) Representative: Griffin, Philippa Jane

(57) **Abstract**

Compositions and methods for performing a template-switching reaction are provided that may include reducing or eliminating concatemerization of the template-switching oligonucleotide (TSO). In some embodiments, the composition may comprise a site-specific nucleic acid cleaving enzyme, a TSO that includes a recognition sequence for a site-specific nucleic acid cleaving enzyme, wherein the TSO has at its 3' end at least one nucleotide that is complementary to one or more non-templated nucleotides added to a templated cDNA strand by the reverse transcriptase, and a site-specific nucleic acid cleaving enzyme that cleaves the TSO at the recognition sequence.

## Description

### CROSS-REFERENCING

This application claims the benefit of: US non-provisional application serial no. 15/240,166, filed August 18, 2016, US provisional application serial no. 62/368,656 filed July 29, 2016, US provisional application serial no. 62/370,469 filed August 3, 2016, US provisional application serial no. 62/370,501 filed on August 3, 2016 and US provisional application serial no. 62/376,493 filed on August 18, 2016, all of which applications are incorporated by reference herein.

### BACKGROUND

In many RNA sequencing (RNA-Seq) methods, the template RNA is amplified. One amplification method takes advantage of the template-switching activity of reverse transcriptase. In such methods, the reverse transcriptase adds one or more non-templated nucleotides onto the 3' end of a templated cDNA and then switches templates to an oligonucleotide that has a 5' sequence that is complementary to the non-templated nucleotides that have been added to the 3' end of the cDNA. Template switching is described in, e.g., US 5,962,272, Zhu et al. (Biotechniques 2001 30:892-897) and in Fig. 1. Template switching methods result in cDNAs that have the complement of a template switching oligonucleotide at their 3' end. The cDNA can be amplified using a primer that hybridizes to the complement of the template switching oligonucleotide along with another primer, e.g., a random primer, an oligo-dT primer, or a gene-specific primer.

One problem with methods that use template-switching is that the polymerase can repeatedly switch between templated and non-templated synthesis at the 3' end. Specifically, the reverse transcriptase may add one or more non-templated nucleotides onto the complement of the switching oligonucleotide, which causes the reverse transcriptase to switch templates from one switching oligonucleotide to another. This results in multiple copies of the complement of the switching oligonucleotide being added onto the 3' end of a cDNA. Such cDNAs, which contain a concatemer of the complement of the template switching oligonucleotide at the 3' end, can cause non-specific background and wasted, and therefore costly, sequencing capacity. This disclosure provides a solution to this problem.

### SUMMARY

Provided herein, among other things, is a composition comprising a reverse transcriptase, a template-switching oligonucleotide comprising a recognition sequence for a site-specific nucleic acid cleaving enzyme, wherein the template-switching oligonucleotide has at its 3' end at least one nucleotide that can hybridize to (i.e., is complementary to) one or more non-templated nucleotides added to a templated cDNA strand by the reverse transcriptase, and the site-specific nucleic acid cleaving enzyme.

Also provided is a method for making cDNA. This method may comprise: obtaining a reaction mix that includes the composition described above and an RNA template; and incubating the reaction mix so as to produce cDNA molecules that comprise, at the 3' end, an added sequence comprising the complement of a sequence of the template-switching oligonucleotide. In this method, during the incubation of (b), the site-specific nucleic acid cleaving enzyme prevents concatemers of the double-stranded form of the template-switching oligonucleotide at the 3' end of the cDNA molecules.

Also provided is a kit. In some embodiments, the kit comprises a reverse transcriptase; a template-switching oligonucleotide comprising a recognition sequence for a site-specific nucleic acid cleaving enzyme, wherein the template-switching oligonucleotide has at its 3' end one or more nucleotides that is complementary to one or more non-templated nucleotides added to a templated cDNA strand by the reverse transcriptase; and a site-specific nucleic acid cleaving enzyme that recognizes the recognition sequence in the template-switching oligonucleotide.

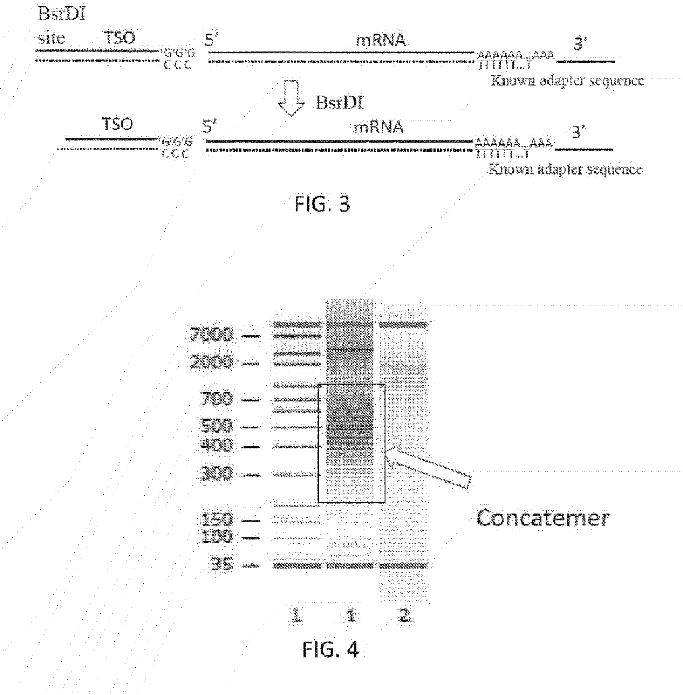

In the present method, the reverse transcriptase switches templates from the RNA to the template-switching oligonucleotide to produce a cDNA that contains the complement of the template-switching oligonucleotide. After the template-switching oligonucleotide has been copied onto the 3' end of the cDNA, the template-switching oligonucleotide and/or the complement of the template-switching oligonucleotide is then cleaved by the site-specific nucleic acid cleaving enzyme. This cleavage prevents a concatemer of the complement of the template-switching oligonucleotide from being formed. The examples section of this disclosure (the result of which are shown in Figs. 4-6) show that this method is very effective in reducing background caused by various lengths of concatemer otherwise formed by template-switching.

In some cases, the entire template-switching reaction can optionally be carried out in a single reaction vessel containing template nucleic acid or cell lysate. This avoids any unnecessary loss of material. One or more of the reagents or the template nucleic acid in the reaction vessel may be free in liquid form (such as free in solution) or may be immobilized on a solid support. For example, optionally, any one or more of the template nucleic acid, the enzymes, the adapter oligo-dT and/or the TSO may be immobilized. In one aspect, the method described herein comprises the step of immobilizing the cDNA product. In one aspect therefore, the cDNA product of RNA reverse transcription is immobilized. Immobilization of the cDNA product of the reverse transcriptase/template-switching is one approach to preventing carryover between reaction steps where the RNA-Seq procedure is performed in two consecutive steps; i.e., reverse transcription and template-switching; and amplification such as PCR for sequencing the amplicon. All or part of a cDNA amplicon can be amplified as desired.

The template switching oligonucleotide may include a label such as a tag or reporter for tracking the completion of concatemer cleavage. Such label may include any molecule that is used by those of ordinary skill in the art to track a DNA dependent reaction (reporter) or immobilize or amplify a DNA dependent reaction (tag). One example of a reporter is a quenching label so that on cleavage, a fluorescent signal occurs which otherwise is quenched by the close proximity of two oligonucleotides. One example of a tag is an aptamer or a biotin molecule or functionally similar molecule that can be used to react with a substrate for immobilizing the cDNA. Optionally, labeling may occur via an agent for immobilizing any one or more of the reagents or products of the reaction as described above.

### BRIEF DESCRIPTION OF THE FIGURES

Some embodiments of the invention may be best understood from the following detailed description when read in conjunction with the accompanying drawings. It is emphasized that, according to common practice, the various features of the drawings are not to scale. Indeed, the dimensions of the various features are arbitrarily expanded or reduced for clarity. Included in the drawings are the following figures.

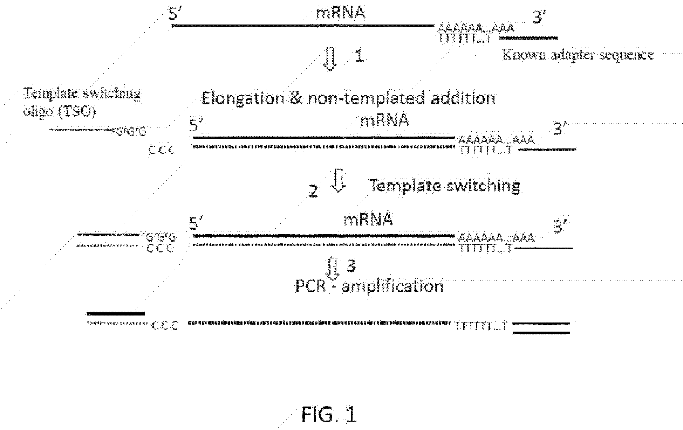

FIG. 1 shows a schematic illustration of cDNA library prep using template-switching.
   (1) A mRNA is shown having a polyA tail. An oligonucleotide cDNA synthesis primer (generally a DNA) with known sequence (Adapter-Oligo dT) hybridizes to the mRNA through the polyA tail and primes the reverse transcriptase. The reverse transcriptase synthesizes a first strand cDNA along the mRNA to the 5' end of the mRNA. In this example, the reverse transcriptase adds a non-templated dCdCdC to the cDNA product at its 3' end.
   (2) A synthetic TSO is designed with a 3'-terminal nucleotide sequence, here exemplified by rGrGrG, which can anneal to the sequence added by non-template addition at the 3' end of the cDNA. The reverse transcriptase then switches from copying the mRNA to copying the TSO.
   (3) PCR primers hybridizing to part or all of the added sequence in the 3' end of the first strand cDNA, and part or all of the sequence at the 5' end of the cDNA that is the complement of cDNA synthesis primer are then added to the reaction to amplify the cDNA.

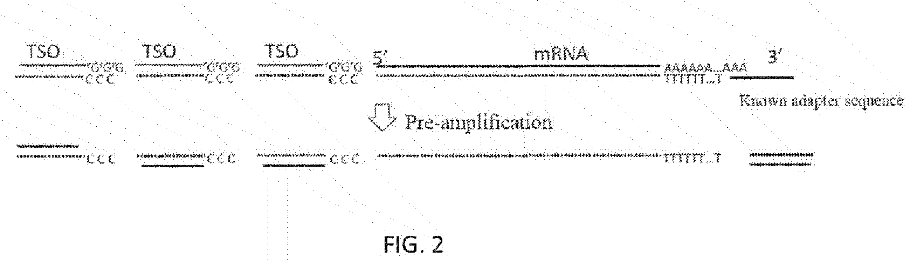

FIG. 2 shows a schematic illustration of concatemer formation that occurs during template-switching by the reverse transcriptase. Once the reverse transcriptase copies to the end of a TSO it adds non-templated nucleotides, a run of dC's in this example, that can hybridize with another TSO that the reverse transcriptase can use as a substrate. In this manner, concatemers of TSO sequences are generated. During PCR, PCR primers anneal to each TSO in the concatemer to produce amplicons of various lengths some of which include amplicons of the concatemer and not the cDNA.
FIG. 3 shows a schematic representation of cleavage of the TSO with a site-specific nucleic acid cleavage enzyme, here exemplified by a restriction enzyme, BsrDI. A cleavage site is designed and introduced into the TSO at its 5' end, and the cleavage enzyme is added to the reaction mixture during the reverse transcription step, which is prior to amplification. The cleavage enzyme removes or prevents concatemers by cleavage of DNA at a position at the 5' end of the TSO. In this example, the reaction conditions include incubating both reverse transcriptase and the cleavage enzyme (BsrDI) at 42°C for 90 mins. The same conditions are effective for both enzymes. Thus, reverse transcription and inhibition of concatemer formation result. Moreover, both enzymes are also inactivated by the same temperature conditions. In this example, the conditions of 70°C for 10 mins were found to be effective.
FIG. 4 shows a virtual gel from a Bioanalyzer^{®} (Agilent, Santa Clara, CA) trace of an mRNA library prepared using template-switching where the TSO contains a recognition sequence for a site-specific nucleic acid cleaving enzyme. In this instance, the site-specific nucleic acid cleaving enzyme is a restriction endonuclease here exemplified by BsrDI.
   Lane 1 shows the results with background contamination caused by concatemers in the absence of a restriction endonuclease, and lane 2 shows the effect of restriction endonuclease cleavage removing background.

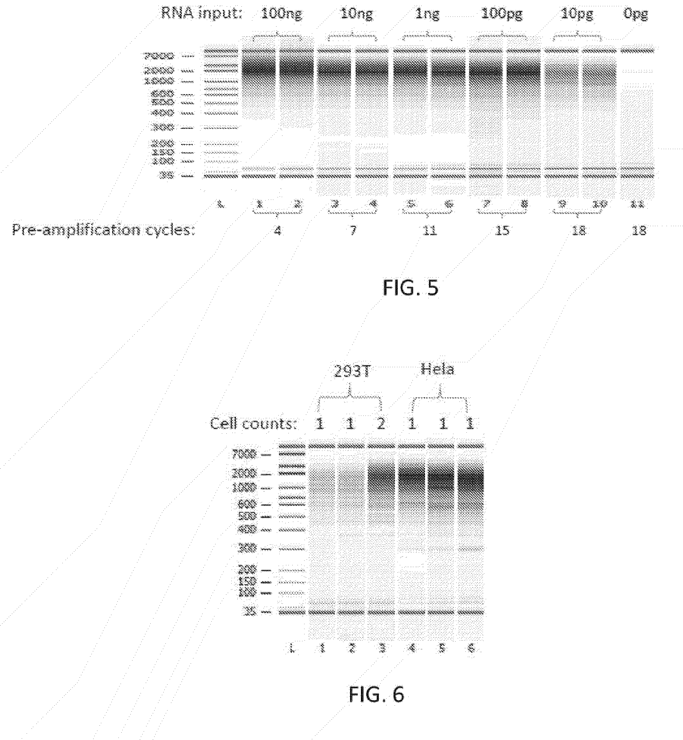

FIG. 5 shows that nucleic acid concentration is not a limiting factor as bands can be detected from as little as 10pg of total RNA (Universal human reference RNA (Agilent, Santa Clara, CA)).
   Lanes 1 and 2 are duplicates of 100ng total RNA input. The cDNA library was amplified 4 cycles.
   Lanes 3 and 4 are duplicates of lOng total RNA input. The cDNA library was amplified 7 cycles.
   Lanes 5 and 6 are duplicates of 1ng total RNA input. The cDNA library was amplified 11 cycles.
   Lanes 7 and 8 are duplicates of 100pg total RNA input. The cDNA library was amplified 15 cycles.
   Lanes 9 and 10 are duplicates of 10pg total RNA input. The cDNA library was amplified 18 cycles.
   Lane 11 is a negative control with no total RNA input.
FIG. 6 shows the results of cDNA libraries with starting material of single cell or two cells (293T or Hela cells).
   Lanes 1, 2 and 3 are cDNA libraries with 1, 1 and 2 293T cells, respectively.
   Lanes 4, 5 and 6 are cDNA libraries with 1, 1, and 1 Hela cell, respectively.

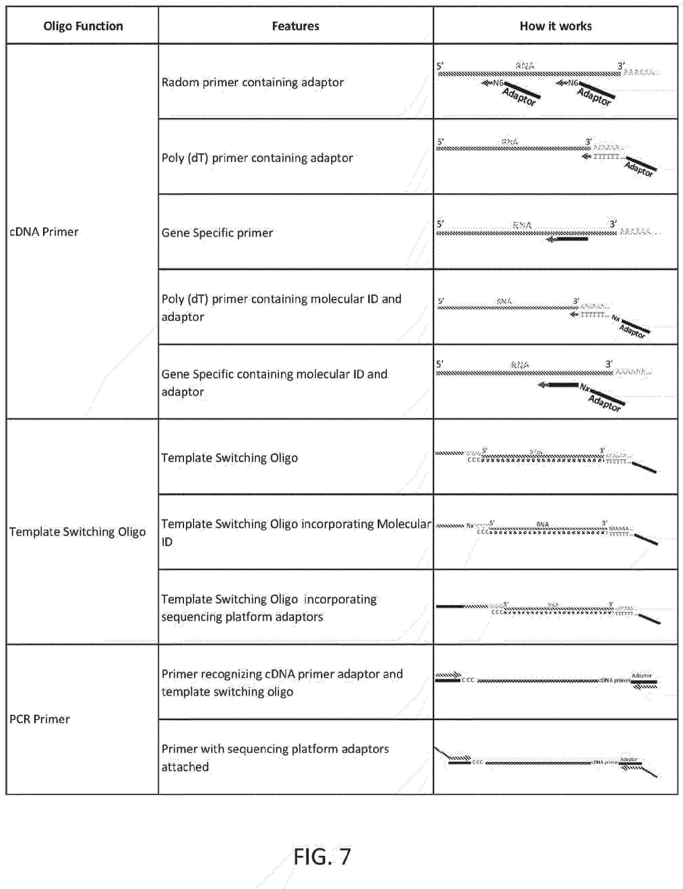

FIG. 7 shows examples of features that may be included in the cDNA synthesis primer (adapter oligo dT) and/or the TSO, including adaptor sequences, primer sequences, sample barcode, unique identifier and oligo dT sequences.
FIG. 8 shows the results of cDNA library prepared within a broad temperature range for a template-switching reaction, in the presence of a site-specific nucleic acid cleavage enzyme. These results demonstrate that the method described herein can be performed at an incubation temperature in the range of 37°C (or lower) to at least 50°C. The reverse transcriptase is a thermostable reverse transcriptase. The site-specific nucleic acid cleaving enzyme is a restriction endonuclease here exemplified by BsrDI.
FIG. 9 shows Bioanalyzer analysis of a cDNA library prepared by template-switching from mRNA where the TSO contains a site-specific nucleic acid cleavage sequence, without and with a crowding agent (Lane 1: without crowding agent, cDNA library yield is 1.0 ng; and Lane 2: with the crowding agent, cDNA library yield is 3.8 ng (5% PEG 8000)).

### DESCRIPTION

Unless defined otherwise herein, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

All patents and publications, including all sequences disclosed within such patents and publications, referred to herein are expressly incorporated by reference.

Numeric ranges are inclusive of the numbers defining the range. Unless otherwise indicated, nucleic acids are written left to right in 5' to 3' orientation; amino acid sequences are written left to right in amino to carboxy orientation, respectively.

The headings provided herein are not limitations of the various aspects or embodiments of the invention. Accordingly, the terms defined immediately below are more fully defined by reference to the specification as a whole.

Unless defined otherwise, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Markham, THE HARPER COLLINS DICTIONARY OF BIOLOGY, Harper Perennial, N.Y. (1991) provide one of skill with the general meaning of many of the terms used herein. Still, certain terms are defined below for the sake of clarity and ease of reference.

It is further noted that the claims may be drafted to exclude any optional element. As such, this statement is intended to serve as antecedent basis for use of such exclusive terminology as "solely", "only" and the like in connection with the recitation of claim elements, or the use of a "negative" limitation.

As used herein, two sequences are said to be "complementary" to one another if they are capable of hybridizing to one another to form an antiparallel, double-stranded nucleic acid structure.

As used herein, the term "solid support" refers to any known substrate which can be used for the immobilization of a binding ligand, an enzyme or an oligonucleotide/polynucleotide sequence by any known method.

As used herein, the term "adapter" refers to a short sequence preferably less than 50 bases that may be synthesized for convenience and is added to one or both ends of a template nucleic acid for purposes of cloning and/or sequencing.

As used herein, the term "reporter" refers to any group incorporated into full-length cDNA by conventional chemical or enzymatic labeling procedures and which can be detected by use of conventional techniques, such as scintillation counting, autoradiography, fluorescence measurement, calorimetric measurement, light emission measurement, and other means known in the art. Examples of reporter groups include radioisotopes, fluorescent, chemifluorescent, chemiluminescent, hapten groups, and others known in the art.

As used herein, the term "nucleotide", in the context of an oligonucleotide, includes any deoxynucleotide or ribonucleotide that is typically found in naturally occurring nucleic acid as well as nucleotide analogs and modified nucleotides that are capable of base pairing with naturally occurring nucleotides. A nucleotide analog is not typically found in DNA or RNA and possesses some additional features which can improve efficiency of the template-switching reaction or improve the usage of anchored cDNA generated. For example, suitable nucleotide analogs include modification in the base or sugar-phosphate backbone, like peptide nucleic acid, inosine, 5-nitroindole deoxyribofuranosyl, 5-methyldeoxycytosine, and 5,6-dihydro-5,6-dihydroxydeoxythymidine. Other nucleotide analogs will be evident to those skilled in the art. Modified nucleotides include methyl or hydroxymethyl nucleotides such as methyl cytosine, methyl guanine, methyl adenine and methyl thymine, 7-deaza-adenine, 7-deaza-guanine, adenine, guanine, cytosine, thymine, uracil, inosine, 8=oxoguanine, 2-deaza-2-thio-guanosine, 2-thio-7-deaza-guanosine, 2-thio- adenine, 2-thio-7-deaza-adenine, isoguanine, 7-deaza-guanine, 5,6-dihydrouridine, 5,6- dihydrothymine, xanthine, 7-deaza-xanthine, hypoxanthine, 7-deaza-xanthine, 2,6 diamino-7-deaza purine, 5-methyl-cytosine, 5-propynyl-uridine, 5-propynyl-cytidine, 2-thio-thymine or 2-thio-uridine. Oligonucleotides or templates or complementary nucleic acid synthesized from the templates may contain nucleotides typically found in DNA or RNA or modified nucleotides or nucleotide analogs.

As used herein, the term "template" includes a polynucleotide sequence that carries genetic code and directs the synthesis of a sequence complementary to it. In one embodiment, an RNA and TSO sequence are the templates for the reverse transcriptase to produce cDNA. Accordingly, the template RNA may be the target in a mixed population of RNA molecules for enrichment. The formed cDNA is then the template for amplification performed by PCR.

As used herein, the term "non-naturally occurring" refers to a composition that does not exist in nature. Any protein described herein may be non-naturally occurring, where the term "non-naturally occurring" refers to a protein that has an amino acid sequence and/or a post-translational modification pattern that is different to the protein in its natural state. For example, a non-naturally occurring protein may have one or more amino acid substitutions, deletions or insertions at the N-terminus, the C-terminus and/or between the N- and C-termini of the protein. A "non-naturally occurring" protein may have an amino acid sequence that is different to a naturally occurring amino acid sequence (i.e., having less than 100% sequence identity to the amino acid sequence of a naturally occurring protein) but that is at least 80%, at least 85%, at least 90%, at least 95%, at least 97%, at least 98% or at least 99% identical to the naturally occurring amino acid sequence. In certain cases, a non-naturally occurring protein may contain an N-terminal methionine or may lack one or more post-translational modifications (e.g., glycosylation, phosphorylation, etc.) if it is produced by a different (e.g., bacterial) cell.

As used herein, the term "mutant" or "variant" protein may have one or more amino acid substitutions relative to a wild-type protein and may include a "fusion" protein. The term "fusion protein" refers to a protein composed of a plurality of polypeptide components that are unjoined in their native state. Fusion proteins may be a combination of two, three or even four or more different proteins. The term polypeptide includes fusion proteins, including, but not limited to, a fusion of two or more heterologous amino acid sequences, a fusion of a polypeptide with: a heterologous targeting sequence, a linker, an epitope tag, a detectable fusion partner, such as a fluorescent protein, β-galactosidase, luciferase, etc., and the like. A fusion protein may have one or more heterologous domains added to the N-terminus, C-terminus, and/ or the middle portion of the protein. If two parts of a fusion protein are "heterologous", they are not part of the same protein in its natural state. In the context of a nucleic acid, the term "non-naturally occurring" refers to a nucleic acid that contains: a) a sequence of nucleotides that is different to a nucleic acid in its natural state (i.e. having less than 100% sequence identity to a naturally occurring nucleic acid sequence), b) one or more non-naturally occurring nucleotide monomers (which may result in a non-natural backbone or sugar that is not G, A, T or C) and/or c) may contain one or more other modifications (e.g., an added label or other moiety) to the 5'- end, the 3' end, and/or between the 5'- and 3'-ends of the nucleic acid.

As used herein, the term "non-naturally occurring" in the context of a preparation, refers to: a) a combination of components that are not combined by nature, e.g., because they are at different locations, in different cells or different cell compartments; b) a combination of components that have relative concentrations that are not found in nature; c) a combination that lacks something that is usually associated with one of the components in nature; d) a combination that is in a form that is not found in nature, e.g., dried, freeze dried, crystalline, aqueous; and/or e) a combination that contains a component that is not found in nature. For example, a preparation may contain a "non-naturally occurring" buffering agent (e.g., Tris, HEPES, TAPS, MOPS, tricine or MES), a detergent, a dye, a reaction enhancer or inhibitor, an oxidizing agent, a reducing agent, a solvent or a preservative that is not found in nature.

As used herein, the term "oligonucleotide" refers to a multimer of at least 10, e.g., at least 15 or at least 30 nucleotides. In some embodiments, an oligonucleotide may be in the range of 15-200 nucleotides in length, or more. Any oligonucleotide used herein may be composed of nucleotides as described above. These include G, A, T, C, and U or nucleotides that are capable of base pairing reliably with a complementary nucleotide. Examples of modified nucleotides include methyl or hydroxymethyl nucleotides such as methyl cytosine, methyl guanine, methyl adenine and methyl thymine, 7-deaza-adenine, 7-deaza-guanine, inosine, 2-deaza-2-thio-guanosine, 2-thio-7-deaza-guanosine, 2-thio- adenine, 2-thio-7-deaza-adenine, isoguanine, 7-deaza-guanine, 5,6-dihydrouridine, 5,6- dihydrothymine, xanthine, 7-deaza-xanthine, hypoxanthine, 7-deaza-xanthine, 2,6 diamino-7-deaza purine, 5-methyl-cytosine, 5-propynyl-uridine, 5-propynyl-cytidine, 2-thio-thymine or 2-thio-uridine. Oligonucleotides may also include nucleotide analogs, as described above, and non-natural linkages. An oligonucleotide may be an LNA^{tm} (Exiqon, Denmark), a peptide nucleic acid (PNA)(PNA Bio, CA), an unlocked nucleic acid (UNA) (TriLink Biotechnology, CA), or an morpholino oligomer (Gene Tools, Oregon). Morpholinos are synthetic molecules that are the product of a redesign of natural nucleic acid structure. Usually 25 bases in length, they bind to complementary sequences of RNA or single-stranded DNA by standard nucleic acid base-pairing, for example. The oligonucleotides used herein may contain natural or non-natural nucleotides or linkages.

As used herein, the term "primer" refers an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process is determined by the sequence of the template polynucleotide. Usually primers are extended by a DNA polymerase. Primers are generally of a length compatible with their use in synthesis of primer extension products, and usually are in the range of between 8 to 100 nucleotides in length, such as 10 to 75, 15 to 60, 15 to 40, 18 to 30, 20 to 40, 21 to 50, 22 to 45, 25 to 40, and so on, more typically in the range of between 18-40, 20-35, 21-30 nucleotides long, and any length between the stated ranges. Primers are usually single-stranded. Primers have a 3' hydroxyl. In one aspect, a primer comprises oligo(dT).

As used herein, the term "crowding agent" refers to an agent that enhances or facilitates molecular crowding. Crowding agents are believed to bind to and tie up water in a solution, allowing other components of the solution to come into closer contact with one another. Crowding agents include a variety of well-known macromolecules, such as polymers, e.g., polyethylene glycol (PEG); Ficoll, such as Ficoll 70; dextran, such as dextran 70; or the like. In general, when PEG is used, a concentration of about 5% (weight/volume) is optimal. However, the amount of PEG can range, e.g., from about 3 to about 7%. Any suitable size of PEG can be used, e.g., ranging from about PEG-200 (e.g., PEG-400, PEG-600, PEG-1000, PEG-1500, PEG-3350, PEG-4000, PEG-6000, or PEG-8000) to about PEG-30,000, or even higher.

As used herein, the term "buffering agent" refers to an agent that allows a solution to resist changes in pH when acid or alkali is added to the solution. Examples of suitable non-naturally occurring buffering agents that may be used in the compositions, kits, and methods of the invention include, for example, Tris, HEPES, TAPS, MOPS, tricine, or MES.

As used herein, the term "full-length complementary DNA or cDNA" is defined as a full-length single-stranded (ss) or double-stranded (ds) cDNA(s), or cDNA fragment(s), which contains the complete sequence information of the template RNA.

As used herein, the term "cDNA library" refers to the whole population of ss or ds cDNAs synthesized from template RNA. The cDNA library can be used directly for different applications known in the art such as for amplification and sequencing, or it can be cloned into any suitable recombinant cloning vehicle. A host can be transformed with the cloning vehicle.

As used herein, the term "template-switching" refers to a process of template-dependent synthesis of a complementary strand by a DNA polymerase using two templates in consecutive order, wherein the two templates are not covalently linked to each other by phosphodiester bonds. The synthesized complementary strand will be a single continuous strand complementary to both templates. In one example, the first template is polyA+RNA and the second template is a TSO. FIG. 1 shows a template-switching reaction involving a mRNA. An oligo dT with a defined adapter sequence hybridizes to the poly (A) 3' end of the mRNA. This serves as a cDNA synthesis primer for a reverse transcriptase that copies the mRNA to form a complementary cDNA strand. The reverse transcriptase commonly travels beyond the 5' end of the mRNA to add non-template nucleotides to the 3' end of the cDNA. If a second primer/adapter (e.g. TSO in FIG. 1) is present having ribonucleotides or deoxyribonucleotides that are complementary to the non-template nucleotides on the cDNA, the reverse transcriptase will continue to extend the cDNA until the end of this adapter-primer, again adding non template nucleotides at the 3' end.

As used herein, the term "concatemer" refers to a DNA molecule that contains multiple copies of the same DNA sequences linked in series. An example is shown in FIG. 2. The concatemer is made up of multiple copies of TSOs where the 3' end of each TSO hybridizes to non template nucleotides added by the transcriptase as it extends the cDNA by template-switching as described above.

As used herein, the term "site-specific nucleic acid cleaving enzyme" refers to an enzyme that is capable of cleaving one or both strands of a double-stranded nucleic acid (such as double-stranded DNA) in a sequence-specific manner, e.g., a sequence-specific endonuclease as defined herein. In one aspect, the site-specific nucleic acid cleaving enzyme cleaves both strands of the double-stranded form of a TSO. A site-specific nucleic acid cleaving enzyme will cleave one or both strands of the double-stranded form of a TSO if the TSO contains a recognition sequence for that enzyme. A site-specific nicking enzyme can cleave one strand of a duplex only. However, if recognition sequences for the nicking enzyme are adjacent but on opposite strands, double-stranded cleavage may result. Further, once a nick is made on one strand, the nucleic acid may cleave on the second strand at the opposite site. Certain nucleases recognize a modified base and nick the nucleic acid at or adjacent to the modified base, e.g., specific repair nucleases that recognize and cleave at 8-oxoguanine uracil (uracil deglycosylase (UDG)). Other nucleases create a double-strand break at those modified bases, e.g., MspJI which recognizes a methyl cytosine and creates a double-strand break of the DNA at a fixed distance from the methyl cytosine. The substrate for the site-specific nucleic acid cleaving enzyme should be double-stranded. However, the enzyme does not need to cleave both strands of the substrate. Rather the enzyme could cleave one or both strands of the substrate.

As used herein, the term "RNA-cDNA hybrid" refers to a product after first-strand cDNA synthesis catalyzed by reverse transcriptase using RNA as a template. "RNA-cDNA hybrid" can be full-length if the cDNA portion includes the complete sequence of the 5'-ends of the template mRNA.

As used herein, the term "5' " refers to any sequence that lies within half of the overall sequence.

As used herein, the term "reverse transcriptase" is defined as any DNA polymerase possessing reverse transcriptase activity which can be used for first-strand cDNA synthesis of template RNA. The reverse transcriptase used in the compositions, kits, and methods may be thermostable, as described herein. Examples of suitable reverse transcriptases (such as thermostable reverse transcriptases) are provided herein.

Before the present invention is described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither, or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described. All publications mentioned herein are incorporated herein by reference to disclose and describe the methods and/or materials in connection with which the publications are cited. It is understood that the present disclosure supersedes any disclosure of an incorporated publication to the extent there is a contradiction.

The present method relies on template switching and exploits an intrinsic property of Moloney murine leukemia virus (M-MLV) reverse transcriptase other reverse transcriptases to switch templates from a template to a unique template switching oligonucleotide (TSO). During first-strand synthesis, upon reaching the 5' end of the RNA template, the terminal transferase activity of the reverse transcriptase adds one or more (e.g., 2, 3, 4, or 5) additional nucleotides (for example,deoxycytidine) to the 3' end of the newly synthesized nucleic acid (e.g., cDNA) strand. The one or more additional nucleotides at the 3' end may be the same or may vary (i.e. nucleotides that differ from each other). These nucleotides function as an anchoring site for the template switching oligonucleotide. Upon base pairing between the template switching oligonucleotide and the additional nucleotides, the reverse transcriptase "switches" template strands, from the cellular nucleic acid to the template switching oligonucleotide, and continues replication to the 5' end of the template switching oligonucleotide. By doing so, the resulting synthesized nucleic acid (e.g. cDNA) contains the complete 5' end of the transcript, and universal sequences of choice are added to the reverse transcription product. Along with tagging of the complementary nucleic acid (e.g. cDNA) at the 3' end by oligo dT primers, this approach makes it possible to efficiently amplify the entire full-length transcript pool in a completely sequence-independent manner. Some template switching oligonucleotides have three riboguanosines (rGrGrG) at its 3' end. Alternatively other nucleotides that are the same or different can be used. The number of nucleotides may vary to correspond with the number of nucleotides added to the template nucleic acid.

In general, a composition is provided that includes: (a) a template-switching oligonucleotide (TSO) comprising a recognition sequence for a site-specific nucleic acid cleaving enzyme , wherein the TSO has at its 3' end at least one nucleotide that can hybridize to, i.e., is complementary with, one or more non-template nucleotides at the 3' end of a cDNA strand of a RNA/cDNA complex; (b) a reverse transcriptase; and (c) the site-specific nucleic acid cleaving enzyme. The TSO, reverse transcriptase, and/or site-specific nucleic acid cleaving enzyme may be as further described herein. In one aspect, the composition further includes an RNA template. Examples of RNA templates are described herein. For example, the RNA template may be purified or come from a cell lysate or from another source. For example, the RNA template may be obtained from a cell lysate of a single cell or a small number of cells (less than 1000 cells, less than 500 cells or less than 100 cells). The RNA template may be total RNA, micro RNA, long non-coding RNA, exome RNA, gene silencing RNA or any other RNA of interest in a sample to be analyzed. For example, the RNA template may be poly A+ RNA. The RNA template may be obtained from a vertebrate, invertebrate, plant or prokaryotic source. For example, the RNA template may be obtained from a mammal. The RNA template may, therefore, be vertebrate (e.g. mammalian), invertebrate, plant or prokaryotic. The RNA template may include a 5' modified GMP cap such as a 7-methylguanosine or an affinity tag-labeled GMP cap.

The TSO may be DNA. In one aspect, the composition includes a cDNA synthesis primer. The cDNA synthesis primer may be an oligo(dT) primer, a random primer, or a transcript-specific primer. In another aspect, the composition includes a cDNA synthesis primer having a 5' tail that does not hybridize to the RNA template. In one aspect, the RNA template has a polyA tail and the cDNA synthesis primer is an oligo(dT) primer such as an adaptor-oligo(dT) primer.

In one aspect, the TSO may be part DNA and part RNA to form a DNA-RNA chimera. The TSO may include an amplification primer sequence, and/or optionally a molecular barcode sequence, between the recognition sequence for the site-specific nucleic acid cleaving enzyme and the at least one 3'-terminal nucleotide(s). In one aspect, the TSO comprises three riboguanine residues at its 3' end.

In one aspect, the composition may further contain a crowding agent. In another aspect, the crowding agent is polyethylene glycol.

In general, a method is provided for making a complementary nucleic acid (e.g., cDNA) that comprises incubating a composition as defined herein with a nucleic acid template (e.g., an RNA template) under conditions (that may include a temperature in the range specified above for a period of at least 30 minutes, e.g., at least 40, at least 50, at least 60, at least 70, at least 80 or at least 90 minutes) so as to produce the complementary nucleic acid ( eg cDNA) molecules having at the 3' end, at least one non-template nucleotide that hybridizes with the 3' end of the TSO. The temperature conditions are also selected so as to be suitable for site-specific nuclease digestion of concatemer of the TSO. As such, the concatemers may be digested as they are being produced in the reverse transcription reaction.

In one aspect, the RNA template is a mRNA template, or any RNA template as defined herein. The at least one non-template nucleotide and the 3' terminal end of the TSO may be as described further herein. In one aspect, the method comprises the initial step of forming a reaction mix by combining the composition with the RNA template, prior to the incubation step. Optionally, the method comprises an additional step of amplifying the cDNA molecules generated in the incubation step to produce amplification products. The amplification may be done by PCR using a first primer and a second primer. The first primer may hybridize to a sequence at the 3' end of the cDNA and/or the second primer may hybridize to a sequence at the 5' end of the cDNA that is the complement of a sequence in the adapter-oligo dT primer. Optionally, the method comprises the step of sequencing the amplification products, following the amplification step.

In another aspect, the reverse transcriptase and/or the site-specific nucleic acid cleaving enzyme may be inactivated such as by incubating the reaction mix for a period of at least 10 minutes at a temperature of at least 10 degree Celsius higher than the temperature that the restriction endonuclease and/or the site-specific nucleic acid cleaving enzyme is active (as described herein). The reverse transcriptase and/or the site-specific nucleic acid cleaving enzyme may be thermostable, as described herein. In one aspect, the RNA template is from a clinical sample such as fetal cells in maternal blood, immune cells responding to a foreign body, or cancer cells from a tumor.

In one aspect of the method, the site-specific nucleic acid cleaving enzyme is a double-strand nucleic acid cleaving enzyme. In one aspect, the double-strand nucleic acid cleaving enzyme is a sequence specific double strand (ds) DNA cleaving enzyme. Examples include: a restriction enzyme, an N-glycosylase for example, Afu Uracil-DNA Glycosylase (NEB), a lyase (N-glycosylase-lyase), an endonuclease for example, Tma endonuclease III, an N-glycosylase/AP-Lyase from Thermotoga maritime that recognizes 5,6 dihydroxythymine and abasic sites in DNA (NEB), Pa-AGOG, an N-glycosylase /AP-Lyase from Pyrobaculum aerophilum that recognizes 8-oxoguanine and abasic sites (see NAR (2004) 32(22):6531-6539), a cas9 ortholog for example, the ortholog obtainable from *Geobacillus stearothermophilus,* and Argonaut ortholog such as PfAgo, an argonaute protein from Pyrococcus furiosus (NAR (2015) 43(10):5120-5129) or TtAgo, an argonaute protein from Thermus thermophiles (Nature (2013) 507:258-261).

In general, a kit is provided that includes: (a) a template-switching oligonucleotide (TSO) comprising a recognition sequence for a site-specific nucleic acid cleaving enzyme, wherein the TSO has at its 3' end at least one nucleotide that is complementary to one or more non-template nucleotides at the 3' end of a cDNA strand of a RNA/cDNA complex; (b) a reverse transcriptase; and (c) the site-specific nucleic acid cleaving enzyme that recognizes the recognition sequence in the TSO. In one aspect, the TSO, the reverse transcriptase, and/or the site-specific nucleic acid cleaving enzyme is as described herein. In one aspect, the kit may further contain a crowding agent. In another aspect, the crowding agent is polyethylene glycol. The components of the kit may be in separate vessels of the same vessel.

In one aspect, the reverse transcriptase is Moloney Leukemia Virus Reverse Transcriptase (M-MLV RT), M-MLV Reverse Transcriptase (M-MLV RT) lacking RNaseH activity (or having reduced RNaseH activity), human T-cell leukemia virus type I (HTLV-I) reverse transcriptase, bovine leukemia virus (BLV) reverse transcriptase, Rous sarcoma virus (RSV) reverse transcriptase, or human immunodeficiency virus (HIV) reverse transcriptase, or a variant thereof. In one respect, the reverse transcriptase is thermostable such as Luna^{®} (New England Biolabs, Inc). In one aspect, the reverse transcriptase is a variant reverse transcriptase that has reduced RNaseH activity as compared to the corresponding wild-type reverse transcriptase, or has no RNaseH activity. In one aspect the reverse transcriptase is a M-MLV RT having reduced or no RNaseH activity.

In one aspect, the site-specific nucleic acid cleaving enzyme is thermostable. In one aspect, the site-specific nucleic acid cleaving enzyme is a restriction endonuclease. In one aspect, the site-specific nucleic acid cleaving enzyme is a thermostable restriction endonuclease,where optionally, the restriction endonuclease is active at a temperature of at least 20°C and as much as 65°C. In one aspect, restriction endonuclease may be active at a temperature of at least 25°C, e.g., at least 30°C, at least 35°C , at least 40°C, at least 45°C, at least 50°C, at least 55°C, at least 60°C, or at least 65°C. In another aspect, the restriction endonuclease is capable of inactivation by incubation for a period of at least 10 minutes at a temperature of at least 10 degree Celsius higher than the temperature that the restriction endonuclease is active. For example, if the restriction endonuclease is active at a temperature of at least 25°C, e.g., at least 30°C, at least 35°C, at least 40°C, at least 45°C, at least 50°C, at least 55°C, at least 60°C, or at least 65°C, the restriction endonuclease is inactivated by incubation for a period of at least 10 minutes at a temperature of at least 35°C, e.g., at least 40°C, at least 45°C, at least 50°C, at least 55°C, at least 60°C, at least 65°C, at least 70°C, or at least 75°C, respectively. The incubation period necessary for inactivation may vary with the restriction endonuclease and the inactivation temperature and may be, for example, at least 10, e.g., at least 15, at least 20, at least 30, or at least 60 minutes. In one aspect, the restriction endonuclease is active at a temperature of about 42°C and is inactivated by incubation at about 70°C for 10 mins.

Reverse transcriptases for use in template-switching include: retroviral reverse transcriptase, retrotransposon reverse transcriptase, retroplasmid reverse transcriptases, retron reverse transcriptases, bacterial reverse transcriptases, group II intron-derived reverse transcriptase, and mutants, variant derivatives, or functional fragments thereof. For example, the reverse transcriptase may be a viral reverse transcriptase, such as M-MLV RT; or the reverse transcriptase may be an invertebrate reverse transcriptase, for example, a Bombyx mori reverse transcriptase (e.g., Bombyx mori R2 non-LTR element reverse transcriptase). In one aspect, the reverse transcriptase is a variant M-MLV RT that has reduced RNaseH activity as compared to a wild-type M-MLV RT. In one embodiment, the reverse transcriptase is thermostable. In one aspect, the reverse transcriptase is active at a temperature of at least 20°C and as much as 65°C. In one aspect, the reverse transcriptase may be active at a temperature of at least 25°C, e.g., at least 30°C, at least 35°C, at least 40°C, at least 45°C, at least 50°C, at least 55°C, at least 60°C, or at least 65°C. In another aspect, the reverse transcriptase is capable of inactivation by incubation for a period of at least 10 minutes at a temperature of at least 10°C higher than the temperature at which the reverse transcriptase is active. For example, if the reverse transcriptase is active at a temperature of at least 25°C, e.g., at least 30°C, at least 35°C, at least 40°C, at least 45°C, at least 50°C, at least 55°C, at least 60°C, or at least 65°C, the reverse transcriptase is inactivated by incubation for a period of at least 10 minutes at a temperature of at least 35°C, e.g., at least 40°C, at least 45°C, at least 50°C, at least 55°C, at least 60°C, at least 65°C, at least 70°C, or at least 75°C, respectively. The incubation period necessary for inactivation may vary with the reverse transcriptase and the inactivation temperature and may be, for example, at least 10 mins, e.g., at least 15, at least 20, at least 30, or at least 60 minutes. In one aspect, the reverse transcriptase is active at a temperature of about 42°C and is inactivated by incubation at about 70°C for 10 mins. Polymerases capable of template-switching that find use in practicing the subject methods are commercially available and include a thermostable reverse transcriptase commercially available from New England Biolabs, Ipswich, MA such as ProtoScript^{®} II or Luna^{®} (US 9,580,698) or a reverse transcriptase commercially available from Life Technologies, Carlsbad, CA (Superscript^{®} II, III, or IV) or SMARTscribe^{™} (Takara Bio, Mountain View, CA) or Maxima H Minus^{™} reverse transcriptase (Thermo Fisher Scientific, Waltham, MA). In certain aspects, a mix of two or more different polymerases or reverse transcriptases are added to the reaction mixture, e.g., for improved processivity, proof-reading, and/or the like (US 2014/0113332, US 9,012,183, Enyeart, et al., Mobile DNA, 5:2 (2014), WO 2014/201273, US 2015/0111789).

Nucleic acid cleavage enzymes used herein are intended to cleave one or both strands of a double stranded nucleic acid, such as one or both strands of a double stranded DNA. Examples include: site-specific endonucleases such as restriction endonucleases described below in more detail and homing endonucleases; and/or guided nucleases such as cas nucleases, argonaute, dicer, transcription activator-like effector nucleases (talens), DNA glycosylases, DNA lyases, structure specific nucleases, or orthologs thereof. DNA cleavage enzymes used herein may be thermostable. Thermostability of a DNA cleavage enzyme such as a restriction endonuclease is described herein. For example, the site-specific endonuclease may be a Tma Endonuclease III or Tth Endonuclease IV, both of that are commercially available from New England Biolabs, Ipswich, MA. The DNA glycosylase may be Afu Uracil-DNA glycosylase (UDG), commercially available from New England Biolabs, Ipswich, MA. The glycosylase may also be archaeal GO-glycosylase (AGOG) from *Pyrobaculum aerophilum* (Sartori, et al., Nucleic Acids Research, 32:22 (2014)). The Argonaut ortholog may be a PfAgo from *Pyrococcus furiosus* (Swarts, et al., Nucleic Acids Research, 43:10 (2015)) or TtAgo from *Thermus thermophiles* (Swarts, et al., Nature, 507:7491 (2014)). The cas9 ortholog may be GeoCas9 from *Geobacillus stearothermophilus* (Harrington, et al., bioRxiv, I 38867 May 16, 2017.). It is preferable that the nucleic acid cleavage enzyme is active within the same temperature range as the reverse transcriptase used in template switching and is also denatured at a similar temperature to that of the reverse transcriptase.

In some embodiments, the DNA cleavage enzyme is a restriction endonuclease with efficient kinetics at 42°C or above. Restriction enzymes with efficient kinetics at 42°C or above, include: ApeKI, Apol, BcIl, BfuAl, BsaBI, BsaJI, BsaWI, BsiEI, BsiHKAI, BsiWI, Bsll, BsmAI, BsmBI, BsmFI, Bsml, BspQI, BsrDI, Bsrl, BssHII, BssKI, BstAPI, BstBI, BstEII, BstNI, BstUI, BstYI, BtgZI, BtsCI, Btsl, BtsMutl, Fatl, Faul, Mwol, Nb. Bsml, Nb.BsrDI, Nt.BspQI, Nt.BstNBI, Phol, PI-PspI, PspGI, Sfil, SmIl, Taql, Tfil, TIil, Tsel, Tsp451, Tsp5091, TspMI, TspRI, Tth111I. (Nb. Bsml, Nb.BsrDI, Nt.BspQI, Nt.BstNBI, are examples of nicking enzymes. Nicking enzymes may be effectively used for concatemer reduction if nicking sites are engineered on each strand adjacent to each other. Alternatively, nicking enzymes can inhibit concatemer formation by destabilizing the concatemer with a nick).

In some embodiments, the DNA cleavage enzyme is a restriction endonuclease with efficient kinetics at 37°C or above. Restriction enzymes with efficient kinetics at 37°C additionally include: Aatll, Accl, Acc65I, Acil, AcIl, Acul, Afel, Aflll, AflIII, Agel, Ahdl, AleI, AIul, AIwl, AlwNI, ApaLI, ApeKI, AscI, Asel, AsiSI, Aval, AvaII, AvrII, BaeGI, BamHI, BanI, BanII, Bbsl, Bbvl, BbvCI, BccI, BceAI, Bcgl, BciVI, BclI, BcoDI, Bfal, BfuCI, Bgll, Bglll, Blpl, BmgBI, Bmrl, Bmtl, Bpml, Bpu10I, BpuEI, Bsal, BsaAI, BsaHI, BsaXI, BseRI, BseYI, Bsgl, BsiWI, BsoBI, Bsp1286l, BspDI, BspEI, BspHI, BspMI, BsrBI, BsrFI, BsrGI, BssSI, BstEII, BstXI, BstZ171, Bsu361, Btgl, Cac8I, Clal, CspCI, CviKI-1, Ddel, Dpnl, Dpnll, Dral, Dralll, Drdl, Eael, Eagl, Earl, Ecil, Eco53KI, EcoNI, EcoO109l, EcoP151, EcoRI, EcoRV, Fnu4HI, Fokl, Fsel, Fspl, FspEI, Haell, Haelll, Hgal, Hhal, HincII, Hindlll, Hinfl, HinP1I, Hpal, Hpall, Hphl, Hpy99I, Hpy166II, Hpy188I, Hpy188III, HpyAV, HpyCH4III, HpyCH4IV, HpyCH4V, I-CeuI, I-SceI, Kasl, Kpnl, LpnPI, Mbol, Mboll, Mfel, Mlul, MIuCI, Mlyl, Mmel, Mnll, Mscl, Msel, Msll, Mspl, MspA1I, MspJI, Nael, Narl, Ncil, Ncol, Ndel, NgoMIV, Nhel, Nlalll, NIaIV, NmeAIII, Notl, Nrul, Nsil, Nspl, PacI, PaeR7I, Pcil, PflFI, PflMI, PI-Scel, Plel, Phol, PluT1, Pmel, Pmll, PpuMI, PshAI, Psil, PspOMI, PspXI, Pstl, Pvul, Pvull, Rsal, Rsrll, Sacl, SacII, SalI, Sapl, Sau3AI, Sau96I, Sbfl, ScaI, ScrFI, SexAI, SfaNI, Sfcl, Sfol, SgrAI, SnaBI, Spel, Sphl, Srfl, Sspl, Stul, Styl, StyD4I, Xbal, Xcml, Xhol, Xmal, Xmnl, Zral, Nb.BbvCI, Nt.Btsl, Nt.Alwl, Nt.BbvCI, Nt.BsmAI.

In some embodiments, the DNA cleavage enzyme is a restriction endonuclease with efficient kinetics at 20°C or above. Restriction enzymes with efficient kinetics at 20°C additionally include: AbaSI, Apal, Bael, BspCNI, CviAII, CviQI, Smal, Swal.

The recognition sequences of each of the above restriction enzymes can be found in ReBASE^{®} (New England Biolabs, Ipswich, MA) or the New England Biolabs 2015-16 catalog incorporated herein by reference.

Examples of RNA templates include mRNAs which have poly A tails. Other templates may include non-polyadenylated RNA, such as long non-coding RNA, small RNAs, and RNA from exosome. RNA templates also include prokaryotic RNA. These RNAs play important roles during development. Malfunctioning of the regulation of any of these RNA regulations can cause pathological conditions. To sequence these RNAs with template-switching, one can use randomized primer (with adapter sequence) or dA-tailing the poly(A)-minus RNA (Turchinovic, et al., RNA Biology, 11(7): 817-828 (2014)). In one aspect, therefore, the method comprises the initial step of preparing the template RNA by adding a dA-tail to a poly(A)-minus RNA.

TSOs are non-naturally occurring or synthetic oligonucleotides. TSOs can have one, or two or more, or three or more (such as three) 3' terminal deoxyribonucleotides or ribonucleotides or a mixture of deoxyribonucleotides and ribonucleotides. These may include a mixture of nucleotides or nucleotide analogues selected from A, G, T, and C. A requirement of these terminal nucleotides is that they are suitable for hybridizing to the one or more non-template nucleotides on the 3' end of the cDNA formed by the reverse transcriptase used in the reaction. In one aspect, the TSO has 3' terminal rG,rG,rG. Some examples of nucleotide modifications and analogues are methylated bases, locked nucleic acids, peptide nucleic acid, or deoxyinosine. In one aspect, the TSO has one, or two or more, or three or more (such as three) 3' terminal methylated bases, locked nucleic acids, peptide nucleic acids, or deoxyinosines. Examples of terminal transferase activities of different reverse transcriptases that add varying nucleotides at the 3' end of the cDNA suitable for template-switching are provided herein by incorporation by reference, for example in Patel, et al., Proc Natl Acad Sci USA, 91(2):549-53 (1994); Golinelli, et al., Virology, 294(1):122-34 (2002); Zajac, et al., PLOS ONE, 8, 12, e85270 (2013); Chen, et al., Biotechniques, 30(3):574-80, 582 (2001) and in Table 1.

**Table 1**

| **RTase** | **Preferred nt** |
|---|---|
| MMLV | C³ |
| HIV-1 | A; A or C |
| HIV-2 | A or C |

In one aspect, therefore, there is provided a method for making cDNA, comprising incubating:
(i) an RNA template, (ii) a composition described herein (comprising a reverse transcriptase, a TSO containing a recognition site for a site-specific nucleic acid cleaving enzyme, and the site-specific nucleic acid cleaving enzyme), and (iii) a cDNA synthesis primer that hybridizes to the 3' end of the RNA template; optionally wherein the RNA template is a mRNA template having a polyA tail and the cDNA synthesis primer comprises oligo dT that hybridizes to the polyA; wherein the cDNA synthesis primer primes the reverse transcriptase so as to copy the RNA template to produce a first strand cDNA strand, and the reverse transcriptase adds one or more non-templated nucleotides to the cDNA product at its 3' end, wherein said one or more non-templated nucleotides are complementary to and hybridize to a nucleotide sequence at the 3' end of the TSO; and wherein, following hybridization of the TSO, the reverse transcriptase extends the 3' end of the cDNA using the TSO as a template; and wherein, during the incubation, the site-specific nucleic acid cleaving enzyme cleaves the TSO, thereby preventing the formation of concatemers of a double-stranded form of the TSO at the 3' end of the cDNA molecules.

In one aspect of the method described herein, the reverse transcriptase adds a non-templated dCdCdC to the cDNA product at its 3' end and the TSO has the 3'-terminal nucleotide sequence rGrGrG.

In one aspect of the method described herein, the reaction mix is incubated at a reaction temperature of 20-65°C (such as at about 42°C), at which the reverse transcriptase and the site-specific nucleic acid cleaving enzyme are active; and then, after the incubation, the reaction mix is incubated at a temperature that is at least 10°C higher than the reaction temperature so as to inactivate the reverse transcriptase and/or the site-specific nucleic acid cleaving enzyme (such as at about 42°C for 10 minutes).

The method may further comprise the step of amplifying the cDNA, such as by PCR, using primers that hybridize to: part or all of the extended sequence at the 3' end of the first strand cDNA, and part or all of the sequence at the 5' end of the cDNA that is the complement of cDNA synthesis primer.

The present method, composition and kits may be used for a variety of applications including, but not limited to, full-length transcriptome profiling from single cells, the analysis of rare RNAs and for the analysis of samples that have limiting amounts of RNA.

### EMBODIMENTS

Embodiment 1. A composition comprising: (a) a reverse transcriptase; (b) a template-switching oligonucleotide comprising a recognition sequence for a site-specific nucleic acid cleaving enzyme, wherein the template-switching oligonucleotide has at its 3' end at least one nucleotide that can hybridize to one or more non-templated nucleotides added to a templated cDNA strand by the reverse transcriptase; and (c) the site-specific nucleic acid cleaving enzyme.

Embodiment 2. The composition of embodiment 1, further comprising a RNA template.

Embodiment 3. The composition of embodiments 1 or 2, wherein the RNA template is total RNA or polyA+ RNA.

Embodiment 4. The composition of any of embodiments 1 to 3, wherein the RNA template is RNA from a mammal.

Embodiment 5. The composition of any of embodiments 1 through 4, wherein the RNA template comprises a 5' modified GMP cap.

Embodiment 6. The composition of embodiment 5, wherein the 5' modified GMP cap is a 7-methylguanosine or an affinity tag-labeled GMP cap.

Embodiment 7. The composition of any of embodiments 1 through 6, further comprising a cDNA synthesis primer, wherein the cDNA synthesis primer is an oligo(dT) primer, a random primer, or a transcript-specific primer.

Embodiment 8. The composition of any of embodiments 1 through 7, further comprising a cDNA synthesis primer having a 5' tail that does not hybridize to the RNA template.

Embodiment 9. The composition of any of embodiment 1 through 8, wherein the template-switching oligonucleotide is a DNA-RNA chimera.

Embodiment 10. The composition of any of embodiments 1 through 9, wherein the template-switching oligonucleotide comprises an amplification primer sequence, and optionally a molecular barcode sequence, between the recognition sequence for the site-specific nucleic acid cleaving enzyme and the at least one nucleotide at its 3' end that is complementary to the non-template nucleotides at the 3' end of the templated cDNA strand.

Embodiment 11. The composition of any of embodiments 1 through 10, wherein the template-switching oligonucleotide comprises three riboguanine residues at its 3' end.

Embodiment 12. The composition of any of embodiments 1 through 11, wherein the reverse transcriptase is selected from the group consisting of Moloney murine leukemia virus (M-MLV), M-MLV reverse transcriptase lacking RNaseH activity, human T-cell leukemia virus type I (HTLV-I) reverse transcriptase, bovine leukemia virus (BLV) reverse transcriptase, Rous sarcoma virus (RSV) reverse transcriptase, human immunodeficiency virus (HIV) reverse transcriptase; and variants thereof.

Embodiment 13. The composition of according to any of embodiments 1 through 12, wherein the reverse transcriptase is thermostable.

Embodiment 14. The composition according to any of embodiments 1 through 13, wherein the site-specific nucleic acid cleaving enzyme cleaves double-stranded DNA,

Embodiment 15. The composition according to any of embodiments 1 through 14, wherein the site-specific nucleic acid cleaving enzyme is thermostable.

Embodiment 16. The composition of embodiments 1 through 14, wherein the site-specific nucleic acid cleavage enzyme is active at a temperature in the range of 20°C to 65°C.

Embodiment 17. The composition of any of embodiments 1 through 15, wherein the site-specific nucleic acid cleaving enzyme is selected from the group consisting of a restriction endonuclease; an N-glycosylase; a lyase (N-glycosylase-lyase); an endonuclease; a cas9 ortholog; and an Argonaut ortholog.

Embodiment 18. The composition of embodiment 16, wherein the site-specific nucleic acid cleaving enzyme is capable of being inactivated after 10 minutes at a temperature at least 10 degree Celsius higher than the reaction temperature for template-switching oligonucleotide cleavage.

Embodiment 19. A method for making cDNA comprising: (a) obtaining a reaction mix comprising the composition of embodiment 1 and an RNA template; and (b) incubating the reaction mix so as to produce cDNA molecules that comprise, at the 3' end, an added sequence comprising the complement of a sequence of the template-switching oligonucleotide; wherein, during the incubation of (b), the site-specific nucleic acid cleaving enzyme prevents concatemers of the double stranded form of the template-switching oligonucleotide at the 3' end of the cDNA molecules.

Embodiment 20. The method of embodiment 19, wherein step (b) comprises incubating the reaction mix at a temperature of 20 °C to 65 °C for a period of at least 30 minutes.

Embodiment 21. The method of embodiments 19 and 20, further comprising, after step (b), inactivating the reverse transcriptase and the site-specific nucleic acid cleaving enzyme by incubating the reaction mix for at least 10 minutes at a temperature at least 10 degrees Celsius higher than the reaction temperature of (b).

Embodiment 22. The method of any of embodiments 19 through 21, further comprising: (c) amplifying the cDNA molecules to produce amplification products.

Embodiment 23. The method of embodiment 22, wherein (c) is performed using a first primer and a second primer, wherein the first primer hybridizes to the added sequence at the 3' end of the cDNA and the second primer hybridizes to the complement of a sequence in the cDNA synthesis primer; and wherein the amplification is performed by PCR.

Embodiment 24. The method of any of embodiments 19 through 23, wherein the method further comprises sequencing the amplification products.

Embodiment 25. The method of any of embodiments 19 through 24, wherein the RNA template is from a clinical sample.

Embodiment 26. The method according to any of embodiments 19 through 25, wherein the reaction mix comprises a crowding agent.

Embodiment 27. The method according to embodiment 26, wherein the crowding reagent is polyethylene glycol.

Embodiment 28. The composition of any of embodiments 1 through 18, comprising a crowding agent.

Embodiment 29. The composition according to embodiment 28, wherein the crowding agent is polyethylene glycol.

Embodiment 30. A kit comprising: (a) a reverse transcriptase; (b) a template-switching oligonucleotide comprising a recognition sequence for a site-specific nucleic acid cleaving enzyme, wherein the template-switching oligonucleotide has at its 3' end one or more nucleotides that is complementary to one or more non-templated nucleotides added to a templated cDNA strand by the reverse transcriptase; and (c) a site-specific nucleic acid cleaving enzyme that recognizes the recognition sequence in the template-switching oligonucleotide.

Embodiment 31. The kit of embodiment 30, further comprising a buffering agent.

Embodiment 32. The kit according to embodiments 30 or 31, further comprising a crowding agent.

Embodiment 33. The kit according to any of embodiments 30-32, wherein the crowding agent is polyethylene glycol.

Following are examples which illustrate procedures for practicing the invention. These examples should not be construed as limiting. All percentages are by weight and all solvent mixture proportions are by volume unless otherwise noted. All references cited herein are incorporated by reference.

### EXAMPLES

### Example 1: Concatemer reduction using a site-specific nucleic acid cleaving enzyme

Single cell isolation can be achieved by serial dilution, flow cytometry, micropipetting, laser capture microdissection, etc. In this example, the sample (293T cells) was trypsinized, washed and stored in PBS buffer and then serially diluted to an appropriate concentration so that one drop of PBS contained only one cell. This could be verified by microscopy. Then the individual cell was put into the 1X lysis buffer with RNase Inhibitor (New England Biolabs, Ipswich, MA) and was incubated at room temperature for 5 minutes. The total RNA in the lysed cell was then analyzed in the following procedure to generate an RNA library. The background was significantly reduced by the use of a site-specific nucleic acid cleaving enzyme to cleave concatemers formed by the reverse transcriptase template-switching by adding multiple TSO sequences. In this example, either single cultured cells or an equivalent amount of total Universal human reference RNA was analyzed.

10 pg (1ul) of total RNA from a single cell or total Universal human reference RNA (Agilent, Santa Clara, CA, #740000), which is equivalent to one cell RNA content, was first annealed to 1 ul immobilized or free Adapter-Oligo dT primer (10 uM stock concentration), 1ul dNTP (10mM stock concentration, New England Biolabs, Ipswich, MA), 1 ul H₂O and incubated at 72°C for 3 minutes. The annealed cooled reaction was incubated with: 0.5ul of ProtoScript II (200 units/ul), 0.25 ul of RNase Inhibitor (40 units/ul), 1ul of BsrDI (5 units/ul, New England Biolabs, Ipswich, MA), 1 ul of Template switching oligo (100uM, stock concentration), 50mM Tris-HCI (final concentration,pH 8.3@25°C), 75mM KCI (final concentration), 9 mM MgCl₂ (final concentration), 2.5mM TCEP (final concentration), 5% PEG8000(final concentration) and H₂O. The total reaction volume is 10 ul.

The template-switching reaction was performed at 42°C for 90 minutes, followed by 72°C for 10 minutes. After the reaction, 1ul PCR primer (10uM, stock concentration) and 2X Q5^{®} Master Mix (New England Biolabs, Ipswich, MA) were added to the reaction (total volume is 50 ul) to perform PCR: denature at 98°C for 30 seconds, 18 cycles of 98°C for 10 seconds, 60°C for 20 seconds and 72°C for 4 minutes followed by 72°C for 10 minutes. The PCR reaction was then purified via AMPure^{®} Beads (Beckman Coulter, Brea, CA) and eluted with 12 ul 0.1XTE. The entire reaction was performed in a single reaction vessel.

The sample was analyzed by digital electrophoresis using a BioAnalyzer 2100. 1ul of the RNA library was loaded to the High Sensitivity DNA chip (Agilent, Santa Clara, CA) in the Bioanalyzer. The results are shown in FIGs. 4-6. The background was significantly reduced in the presence of the site-specific nucleic acid cleaving enzyme due to reduction of concatemer formation.

### Example 2: Concatemer reduction using a site-specific double strand nucleic acid cleaving enzyme within broad temperature range for template-switching reaction

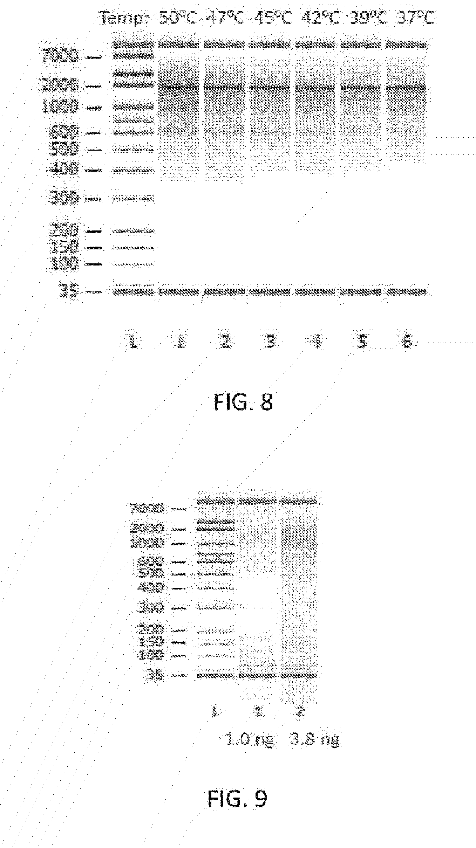

100 pg (1ul) of total Universal human reference RNA was first annealed to 1 ul Adapter-Oligo dT primer (10 uM stock concentration), 1ul dNTP (10mM stock concentration, NEB, Ipswich, MA, #N0447), 1 ul H₂O at 72°C for 3 minutes followed by 4°C. Then, the annealed mix was incubated with the following components: 0.5ul of Luna^{®} (200 units/ul,New England Biolabs, Ipswich, MA), 0.25 ul of RNase Inhibitor (40 units/ul), 1ul of BsrDI (5 units/ul, New England Biolabs, Ipswich, MA), 1ul of TSO (100uM stock concentration), 50mM Tris-HCI (final concentration, pH 8.3@25°C), 75mM KCl(final concentration), 9mM MgCl₂(final concentration), 2.5mM TCEP(final concentration), 5% PEG8000(final concentration) and H₂O. The total reaction volume is 10 ul.The template-switching reaction was performed at 50°C, 47°C, 45°C, 42°C, 39°C or 37°C for 90 minutes, followed by 72°C for 10 minutes. After the reaction, 1ul PCR primer (10uM) and 2X Q5 master mix (New England Biolabs, Ipswich, MA) were added to the reaction to perform PCR. The total volume for PCR reaction was 50 ul. The PCR reaction was then purified via Ampure^{®} Beads (Beckman Coulter, Brea, CA) and eluted with 12 ul of 0.1X TE. The sample was analyzed by digital electrophoresis using a BioAnalyzer 2100. 0.1ul of the RNA library was loaded to the High Sensitivity DNA chip (Agilent, Santa Clara, CA) in the Bioanalyzer. As shown in FIG. 8, the background was significantly reduced in the presence of the site-specific nucleic acid cleaving enzyme within broad temperature range for template-switching reaction.

### Example 3: Use of a crowding agent with a site-specific double-strand nucleic acid cleaving enzyme to reduce background signal

10 pg (1ul) of total RNA (Universal human reference RNA, Santa Clara, CA, #740000) was first annealed with 1 ul Adapter-Oligo dT primer (10 uM stock concentration), 1ul dNTP (10mM stock concentration NEB, Ipswich, MA, #N0447), 1 ul H₂O at 72°C for 3 minutes followed by 4°C. Then, the annealed mix was incubated with the following components: 0.5ul of ProtoScript II (200 units/ul), 0.25 ul of RNase Inhibitor (40 units/ul), 1ul of BsrDI (5 units/ul, New England Biolabs), 1ul of template-switching oligo (100uM, stock concentration), 50mM Tris-HCI (final concentration)pH 8.3@25°C), 75mM KCI (final concentration), 9mM MgCl₂(final concentration), 2.5mM TCEP(final concentration), 5% PEG8000 (final concentration) and H₂O. The total reaction was 10 ul. The template-switching reaction was performed at 42°C for 90 minutes, followed by 72°C for 10 minutes. After the reaction, 1ul PCR primer (10uM, stock concentration) and 2X Q5 master mix were added to the reaction (the total reaction volume was 50 ul) to perform PCR followed by 72°C for 10 minutes. The PCR reaction was then purified via Ampure Beads. As shown in the FIG. 9, PEG enhanced the cDNA library yield.

## Claims

1. A composition comprising:
(a) a reverse transcriptase;
(b) a template-switching oligonucleotide comprising a recognition sequence for a site-specific nucleic acid cleaving enzyme, wherein the template-switching oligonucleotide has at its 3' end at least one nucleotide that can hybridize to one or more non-templated nucleotides added to a templated cDNA strand by the reverse transcriptase; and
(c) the site-specific nucleic acid cleaving enzyme.

2. The composition of claim 1, further comprising a RNA template; such as wherein the RNA template is total RNA or polyA+ RNA; and/or wherein the RNA template is RNA from a mammal; and/or wherein the RNA template comprises a 5' modified GMP cap, such as wherein the 5' modified GMP cap is a 7-methylguanosine or an affinity tag-labeled GMP cap.

3. The composition of claim 1 or claim 2, further comprising a cDNA synthesis primer, wherein the cDNA synthesis primer is an oligo(dT) primer, a random primer, or a transcript-specific primer; and/or wherein the cDNA synthesis primer has a 5' tail that does not hybridize to the RNA template.

4. The composition of any of claims 1 through 3, wherein the template-switching oligonucleotide is a DNA-RNA chimera; and/or wherein the template-switching oligonucleotide comprises an amplification primer sequence, and optionally a molecular barcode sequence, between the recognition sequence for the site-specific nucleic acid cleaving enzyme and the at least one nucleotide at its 3' end that is complementary to the non-template nucleotides at the 3' end of the templated cDNA strand; and/or wherein the template-switching oligonucleotide comprises three riboguanine residues at its 3' end.

5. The composition of any of claims 1 through 4, wherein the reverse transcriptase is selected from the group consisting of Moloney murine leukemia virus (M-MLV), M-MLV reverse transcriptase lacking RNaseH activity, human T-cell leukemia virus type I (HTLV-I) reverse transcriptase, bovine leukemia virus (BLV) reverse transcriptase, Rous sarcoma virus (RSV) reverse transcriptase, human immunodeficiency virus (HIV) reverse transcriptase; and variants thereof; and/or wherein the reverse transcriptase is thermostable.

6. The composition according to any of claims 1 through 5, wherein the site-specific nucleic acid cleaving enzyme cleaves double-stranded DNA.

7. The composition according to any of claims 1 through 6, wherein the site-specific nucleic acid cleaving enzyme is thermostable; or
wherein the site-specific nucleic acid cleavage enzyme is active at a temperature in the range of 20°C to 65°C; optionally wherein the site-specific nucleic acid cleaving enzyme is capable of being inactivated after 10 minutes at a temperature at least 10°C higher than the reaction temperature for template-switching oligonucleotide cleavage;
and/or wherein the site-specific nucleic acid cleaving enzyme is selected from the group consisting of a restriction endonuclease; an N-glycosylase; a lyase (N-glycosylase-lyase); an endonuclease; a cas9 ortholog; and an Argonaut ortholog.

8. The composition of any of claims 1 through 7, comprising a crowding agent, such as polyethylene glycol.

9. A method for making cDNA comprising:
(a) obtaining a reaction mix comprising the composition of claim 1 and an RNA template; and
(b) incubating the reaction mix so as to produce cDNA molecules that comprise, at the 3' end, an added sequence comprising the complement of a sequence of the template-switching oligonucleotide; optionally wherein the incubation is at a temperature of 20°C to 65°C for a period of at least 30 minutes;
wherein, during the incubation of (b), the site-specific nucleic acid cleaving enzyme prevents concatemers of the double-stranded form of the template-switching oligonucleotide at the 3' end of the cDNA molecules.

10. The method of claim 9, further comprising, after step (b), inactivating the reverse transcriptase and the site-specific nucleic acid cleaving enzyme by incubating the reaction mix for at least 10 minutes at a temperature at least 10°C higher than the reaction temperature of (b).

11. The method of claim 9 or claim 10, further comprising: (c) amplifying the cDNA molecules to produce amplification products; such as wherein (c) is performed using a first primer and a second primer, wherein the first primer hybridizes to the added sequence at the 3' end of the cDNA and the second primer hybridizes to the complement of a sequence in the cDNA synthesis primer; and wherein the amplification is performed by PCR.

12. The method of any of claims 9 through 11, wherein the method further comprises sequencing the amplification products.

13. The method of any of claims 9 through 12, wherein the RNA template is from a clinical sample.

14. The method according to any of claims 9 through 13, wherein the reaction mix comprises a crowding agent, such as polyethylene glycol.

15. A kit comprising:
(a) a reverse transcriptase;
(b) a template-switching oligonucleotide comprising a recognition sequence for a site-specific nucleic acid cleaving enzyme, wherein the template-switching oligonucleotide has at its 3' end one or more nucleotides that is complementary to one or more non-templated nucleotides added to a templated cDNA strand by the reverse transcriptase; and
(c) a site-specific nucleic acid cleaving enzyme that recognizes the recognition sequence in the template-switching oligonucleotide;
and optionally further comprising a buffering agent and/or a crowding agent, such as polyethylene glycol.
